**Europäisches Patentamt**

**European Patent Office**

**Office européen des brevets**

(19)

(11) **EP 1 421 895 A1**

(12) **EUROPÄISCHE PATENTANMELDUNG**

| | |
|---|---|
| (43) Veröffentlichungstag:<br>**26.05.2004 Patentblatt 2004/22** | (51) Int Cl.⁷: **A61B 5/00** |

(21) Anmeldenummer: **03023889.3**

(22) Anmeldetag: **21.10.2003**

| | |
|---|---|
| (84) Benannte Vertragsstaaten:<br>**AT BE BG CH CY CZ DE DK EE ES FI FR GB GR HU IE IT LI LU MC NL PT RO SE SI SK TR**<br>Benannte Erstreckungsstaaten:<br>**AL LT LV MK**<br><br>(30) Priorität: **25.11.2002 DE 10255013** | (71) Anmelder: **SIEMENS AKTIENGESELLSCHAFT**<br>**80333 München (DE)**<br><br>(72) Erfinder:<br>• **Pfister, Marcus, Dr.**<br>  **91058 Erlangen (DE)**<br>• **Scholz, Bernhard, Dr.**<br>  **91336 Heroldsbach (DE)** |

(54) **Verfahren und Vorrichtung zur Lokalisierung von Licht emittierenden Bereichen**

(57) Ein Verfahren und eine Vorrichtung zur Durchführung des Verfahrens zur Lokalisierung von Bereichen (2) in einem biologischen Gewebeabschnitt (1), die zumindest während der Untersuchung eine vom Gewebeabschnitt (1) verschiedene Fluoreszenzeigenschaft aufweisen, aufgrund derer bei einer Bestrahlung mit Licht (33) einer ersten Wellenlänge Licht (34) einer anderen Wellenlänge emittiert wird, mit den Schritten:

a) Anlegen einer Folge von fluoreszenzanregenden Lichtsignalen (33) an unterschiedlichen Orten auf dem Gewebeabschnitt (1),

b) Messen (21) von Fluoreszenzlicht (34) an mehreren Messorten auf einer Oberfläche des Gewebeabschnittes (1), die sich aufgrund der Lichtsignale dort einstellen,

c) Bestimmen von frequenzunabhängigen Signalanteilen in den Antwortsignalen und Weiterverarbeitung (23,24) der frequenzunabhängigen Signalanteile zu Eingabewerten eines Lokalisationsschritts (25),

d) Modellieren des Gewebeabschnitts (1) und Bestimmen eines Satzes von Führungsfeldern (22),

e) Transformierung (28) der Führungsfelder (22), so dass im Lokalisierungsschritt (25) die frequenzunabhängigen Signalanteile mit den transformierten Führungsfeldern verglichen werden und dass der Ort (26) der transformierten Führungsfelder, die die frequenzunabhängigen Signalanteile am besten wiedergeben, als Ort des zu lokalisierenden Bereichs (2) ausgegeben wird.

FIG 2

EP 1 421 895 A1

**Beschreibung**

Die Erfindung betrifft ein Verfahren sowie eine Vorrichtung zur Durchführung des Verfahrens zur Lokalisierung von Bereichen, beispielsweise fokalen Läsionen, in einem biologischen Gewebeabschnitt, die zumindest während der Untersuchung eine vom Gewebeabschnitt verschiedene Fluoreszenzeigenschaft aufweisen, aufgrund derer bei einer Bestrahlung mit Licht einer ersten Wellenlänge Licht einer anderen Wellenlänge emittiert wird.

In Umar Mahmood et al., "Near Infrared Optical Imaging of Protease Activity for Tumor Detection", Radiology 213:3, 866-870 (1999) ist beschrieben, dass fluoreszierende metabolische Marker

- sich entweder ausschließlich in bestimmten Regionen, beispielsweise Tumoren, Entzündungen oder anderen bestimmten Krankheitsherden, anreichern, oder
- zwar überall im Körper verteilt sind, aber nur speziell in bestimmten Regionen, beispielsweise durch tumorspezifische Enzymaktivitäten und durch zusätzliche Bestrahlung mittels Licht, aktiviert werden.

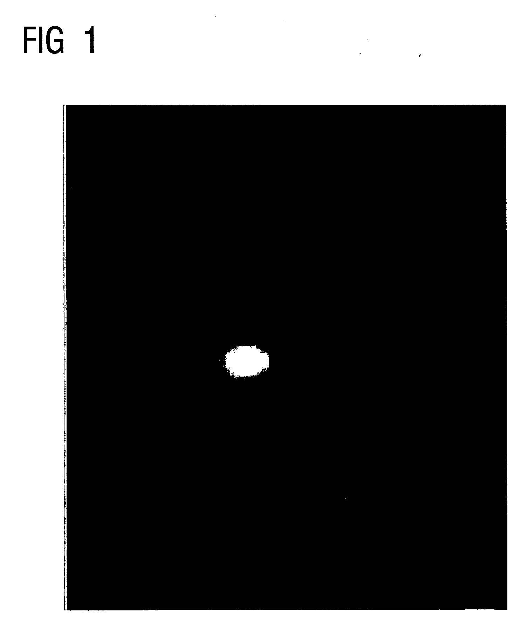

Dieses Prinzip der optischen Fluoreszenz-Bildgebung wird anhand der Figur 1 verdeutlicht, bei dem ein Tumor bei der Beleuchtung mit NIR-Licht (Licht im nahen Infrarotbereich) sichtbar wird, nachdem der Maus ein Marker verabreicht wurde, dessen Fluoreszenzeigenschaften durch bestimmte Enzyme freigeschaltet werden.

Die Erkennung eines Tumors oder einer anderen markierten Region geschieht dann über Bestrahlung der Region mit Licht in der speziellen Anregungswellenlänge des Fluoreszenzfarbstoffes und Detektion des emittierten Lichtes in der entsprechenden Emissionswellenlänge des Fluorophors. Diese Marker können bei Zulassung für den Humanbereich beispielsweise bei der Krebsfrüherkennung eingesetzt werden.

Durch Anregen des Farbstoffes mit mindestens einem zeitlich veränderlichen Anregungslichtsignal, beispielsweise durch zeitliche Variation von Einstrahlungsort und/oder Lichtwellenlänge und/oder Intensitätsmodulation des Anregungslichtes, können Daten z.B. mittels CCD oder Photomultiplier, Photonenfluss auf der Oberfläche des zu untersuchenden Gewebeabschnitts, wie z.B. der weiblichen Brust, an verschiedenen Messpunkten zu verschiedenen Variationen gewonnen werden. Auf diese Weise erhält man variations- und ortsabhängige - d.h. räumlich zweidimensionale - Messdaten. Im Falle von M Messdaten zu $N$ Variationen sind dies $M \times N$ Daten.

Aus diesen Daten können mittels des hier dargestellten Rechenverfahrens räumlich begrenzte Läsionen - wie z.B. fokale fluoreszierend markierte Tumore - mit zum umliegenden Gewebe unterschiedlichen Fluoreszenzeigenschaften - dreidimensional geortet werden.

Die Methode ist vorgesehen bei Krebs- (Screening-) Untersuchungen der

- Brust
- Lymphknoten
- Schilddrüse
- Prostata
- intraoperativen Anwendungen

sowie allen oberflächennahen Organen, die im Bereich der Eindringtiefe von Licht liegen und Karzinome (oder andere Krankheiten) entwickeln, für die (jetzt oder zu einem zukünftigen Zeitpunkt) entsprechende Fluoreszenzmarker existieren.

Zur Fluoreszenzrekonstruktion bzw. Lokalisation existieren verschiedene Ansätze.

Britton Chance schlug ein Verfahren zur Lokalisation von fluoreszierenden Absorbern in homogenem Medium vor, sogenannte Phased Arrays. Dieses Verfahren lokalisiert fluoreszierende Inhomogenitäten (Spots) lediglich in absolut homogenen Medien, d.h. Medien mit homogenen Lichtabsorptions- und Streueigenschaften, wie sie in der Anwendung kaum vorkommen, und bietet keinerlei Information über die Tiefe, in der sich der Spot befindet.

Ansonsten wurden verschiedenen Verfahren zur Fluoreszenz-Rekonstruktion vorgeschlagen. Bei der Rekonstruktion wird die komplette Fluoreszenzaktivität im gesamten (meist diskretisierten) Medium ermittelt (ähnlich wie bei nuklearmedizinischen Methoden), während bei der Lokalisation äusschließlich die sich aus dem Hintergrund hervorhebenden Bereiche gesucht werden. Rekonstruktionsverfahren beruhen somit auf der (oft iterativen) Lösung großer Gleichungssysteme und sind somit, im Gegensatz zur hier vorgeschlagenen, in Echtzeit arbeitenden Lokalisation, sehr zeitaufwendig. Die Rekonstruktionsverfahren gehen des weiteren überwiegend davon aus, dass das zu untersuchende Medium (ähnlich wie bei der Computertomographie) von einem Ring von Lichtquellen und Detektoren umgeben ist.

Einige der bekannten Verfahren sind hier zusammengefasst beschrieben:

Der US 6,304,771 bzw. der US 5,865,754 sind die Tomographie mit frequenzmoduliertem Licht zu entnehmen. Das Rechenverfahren benötigt eine Rekonstruktionszeit von 5 Min. auf einem 1GHz Pentium-Rechner bzw. 45 Min. auf einer SUN Sparc 2 Workstation. In der WO 02/ 41760 A2 ist die Tomographie mit Licht beschrieben, die ebenfalls eine Rekonstruktionszeit von 5 Min. auf einem 1GHz Pentium-Rechner benötigt.

Alle diese beschriebenen Verfahren zeichnen sich durch einen hohen Rechenaufwand bei relativ kleinen Rekonstruktionsvolumina aus, eine Berechnung in Echtzeit ist nicht möglich.

Die Erfindung geht von der Aufgabe aus, bei einem Lokalisierungsverfahren der eingangs genannten Art sowie einer Vorrichtung zur Durchführung des Verfahrens die Lokalisierungsgenauigkeit beispielsweise mittels Fluorophore fluoreszierend markierter Tumore zu erhöhen sowie eine Auswertung in tiefen Gewebeschichten zu ermöglichen und den Rechenaufwand und als Folge davon die Rechenzeit drastisch zu senken.

Die Aufgabe wird erfindungsgemäß für das Verfahren durch die Merkmale des Patentanspruches 1 gelöst. Durch das erfindungsgemäße Verfahren kann das Problem der Lokalisierung fluoreszierender Objekte in optisch trüben Medien schnell gelöst werden. Weiterhin wird durch die Variation des Anregungsortes die Genauigkeit erhöht.

Die Lichtemission von Gewebeabschnitten, in welchen Fluoreszenzmarker angereichert sind, wird durch Einstrahlung von Laserlicht geeigneter Wellenlänge angeregt. Fluoreszenzlicht kann dann an der naheliegenden Hautoberfläche gemessen werden.

Um Orte und optische Parameter markierter Gewebeabschnitte zu bestimmen wird

- eine Folge von Fluoreszenzanregungen von der Oberfläche, beispielsweise von verschiedenen Orten mit verschiedenen Modulationsfrequenzen (einschließlich Frequenz Null), in das Gewebe eingestrahlt, und dann
- das Fluoreszenzlicht mit einer oder mehreren Anordnungen geeigneter, auf der Oberfläche verteilter Lichtsensoren gemessen, um somit zweidimensionale Messwertverteilungen zu erhalten, welche von der Art der Anregung abhängen.

Es hat sich als vorteilhaft erwiesen, wenn zur Erzeugung der verschiedenen Fluoreszenzeigenschaften die Bereiche mit fluoreszierenden Markern (Fluorophore) markiert werden.

Die Ortsauflösung wird erhöht, wenn die fluoreszenzanregenden Lichtsignalen mit verschiedenen Modulationsfrequenzen erzeugt und in den Gewebeabschnitt eingestrahlt werden.

In vorteilhafter Weise können die fluoreszenzanregenden Lichtsignalen durch Laserlicht geeigneter Wellenlänge eingestrahlt werden.

Zweckmäßigerweise werden die Führungsfelder zunächst normiert und dann transformiert, wobei die Führungsfelder zu orthogonalen Führungsfeldern transformiert werden können. Weiterhin können die orthogonalen Führungsfelder mittels einer Singulärwertzerlegung aus den Führungsfeldern bestimmt werden.

Erfindungsgemäß können die optischen Parameter durch Referenzmessungen bei nicht-fluoreszenzanregenden Wellenlängen mittels Schätzverfahren bestimmt werden.

Die Aufgabe wird erfindungsgemäß für eine Vorrichtung zur Durchführung des Verfahrens durch die Merkmale des Patentanspruches 8 gelöst.

Ein Messsystem kann beispielsweise 8x8 regulär, auf einer ebenen Messfläche angeordnete Lichtsensoren enthalten. Es kann jedoch vorteilhaft sein, mit mehreren solcher planarer Systeme gleichzeitig zu messen. So können beispielsweise zwei Anordnungen von Lichtsensoren vorgesehen sein, die beidseitig des zu untersuchenden Gewebeabschnittes anlegbar sind. Dadurch können bei Messungen an der weiblichen Brust zwei Messflächen auf entgegengesetzten Seiten der Mamma angelegt werden. Eine vorteilhafte Ausgestaltung ist die integrierte Anordnung der Messflächen in Anpressplatten eines Röntgen-Mammographie-Gerätes.

Im allgemeinen können beliebig gekrümmte bzw. krümmbare oder flexible Messflächen mit beliebiger Anordnung von Lichtsensoren zum Einsatz kommen.

Die Erfindung ist nachfolgend anhand von in der Zeichnung dargestellten Ausführungsbeispielen näher erläutert. Es zeigen:

Figur 1     Aufnahme zur Erläuterung des Prinzips der optischen Fluoreszenz-Bildgebung,

Figur 2     in einer Übersichtsdarstellung die wesentlichen Komponenten eines Gerätes zum Lokalisieren und Klassifizieren einer fokalen Läsion in einem Gewebeabschnitt,

Figur 3     die wesentlichen Verfahrensschritte zum Lokalisieren einer fokalen Läsion,

Figur 4     erfindungsgemäßer Applikator mit 8x8 Sensoren sowie 8 neben der Messfläche angeordneten Lichtquellen zur Erzeugung des Anregungslichtes,

Figur 5     ein Doppelsystem mit zwei gegenüberliegenden Applikatoren,

Figur 6     eine zweidimensionale Messwertverteilung der Konfiguration 1 für die ersten vier Anregungsorte,

Figur 7 ein Singulärwertspektrum der Konfiguration 1,

Figur 8 Basismaps der Konfiguration 1,

Figur 9 Lokalisierungsfunktionen zweier Fluorochrom-markierten Läsionen,

Figur 10 die Lokalisierung unterschiedlich tiefer Läsionen mit einem planaren Messsystem und

Figur 11 die Lokalisierung unterschiedlich tiefer Läsionen mit zwei gegenüberliegenden planaren Messsystemen.

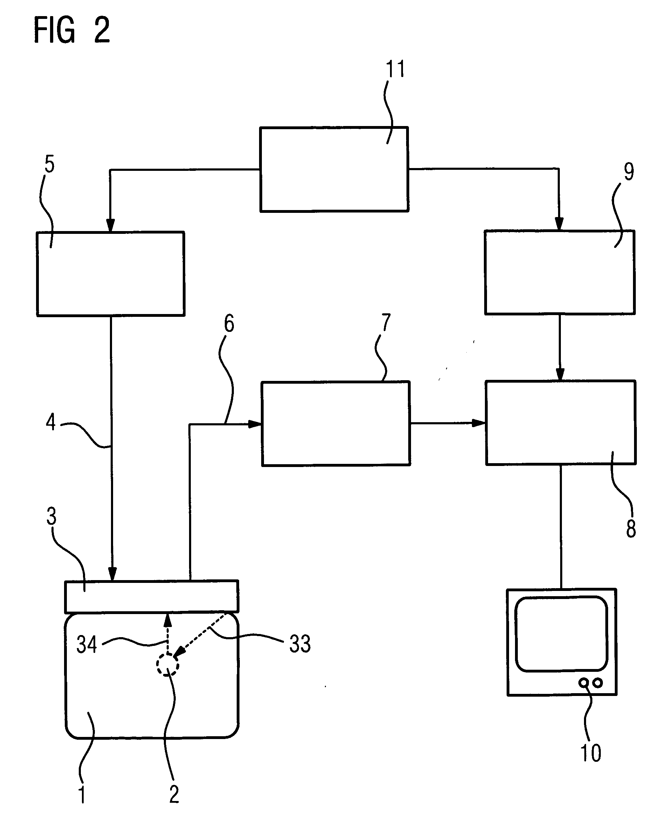

Die Übersichtsdarstellung in Figur 2 zeigt eine Mess- und Auswerteanordnung, womit ein begrenztes, in einem biologischen Gewebeabschnitt 1 angeordnetes Raumgebiet 2 lokalisiert und identifiziert werden kann. Dabei ist vorausgesetzt, dass das Raumgebiet 2 eine vom übrigen Gewebeabschnitt 1 verschiedene Fluoreszenzeigenschaft besitzt. Diese Voraussetzungen sind ausreichend gut erfüllt, wenn es sich bei dem biologischen Gewebeabschnitt 1 um eine weibliche Brust und bei dem begrenzten Raumgebiet 2 um einen Tumor handelt, dem beispielsweise ein fluoreszierender metabolischer Marker zugeführt wurde, dessen Fluoreszenzeigenschaften durch bestimmte Enzyme freigeschaltet werden

Zur Messanordnung gehört ein Applikator 3 mit einer Vielzahl von räumlich verteilt angeordneten Photosensoren sowie daneben in einer Zeile angeordneten Laserdioden, wie dies noch ausführlich beschrieben wird.

Die Erkennung eines Tumors oder einer anderen markierten Region geschieht dann über Bestrahlung der Region mit Licht der Laserdioden in der speziellen Anregungswellenlänge des Fluoreszenzfarbstoffes und Detektion des emittierten Lichtes durch die Photosensoren in der entsprechenden Emissionswellenlänge des Fluorophors.

Die Photosensoren und Laserdioden des Applikators 3 sind zum einen über elektrische Verbindungsleitungen 4 mit einer elektrischen Steuervorrichtung 5 und zum anderen über elektrische Verbindungsleitungen 6 mit einer Messwertaufbereitung 7 verbunden.

Mit Hilfe der Steuervorrichtung 5 werden dem biologischen Gewebeabschnitt 1 über eine Anzahl von K Laserdioden, wobei $1 \leq K \leq M$ ist, Impulse von NIR-Licht zugeführt, um dort eine einen gegebenenfalls vorhanden markierten Tumor zum Fluorizieren anzuregen.

Zur Lokalisierung und Identifizierung von räumlich begrenzten Gebieten 2 wird das von den Gebieten 2 emittierte Licht auf der Oberfläche des Gewebeabschnittes 1 an M Orten mit Photosensoren gemessen und einer Auswertung zugeführt.

Die Messwertaufbereitung 7 umfasst z.B. Messverstärker, Filter und Analog-Digital-Wandler. Die Messwertaufbereitung 7 ist mit einem oder mehreren Dateneingängen eines elektronischen Rechners 8 verbunden. Neben den Messwerten wird dem Rechner ein Modell 9 des Gewebeabschnitts 1 zur Verfügung gestellt, mit dessen Hilfe die oben erwähnten fluoreszierenden Gebiete 2 lokalisiert und identifiziert werden, wie weiter unten noch beschrieben ist. Das Ergebnis, z.B. in Form einer graphischen Darstellung der Anatomie des Gewebeabschnitts, worin der Ort der Lichtquellen und damit der Raumgebiete 2 markiert ist, erfolgt über einen Monitor 10. Da die Berechnung unter anderem von dem Modell 9 und dem Ort der Beleuchtung bestimmt ist, ist eine übergeordnete Eingabe und Steuerung 11 vorgesehen, mit der die Anzahl und der Ort der Photosensoren sowie der Laserdioden, der Wert der Frequenz und das Modell vorgegeben werden.

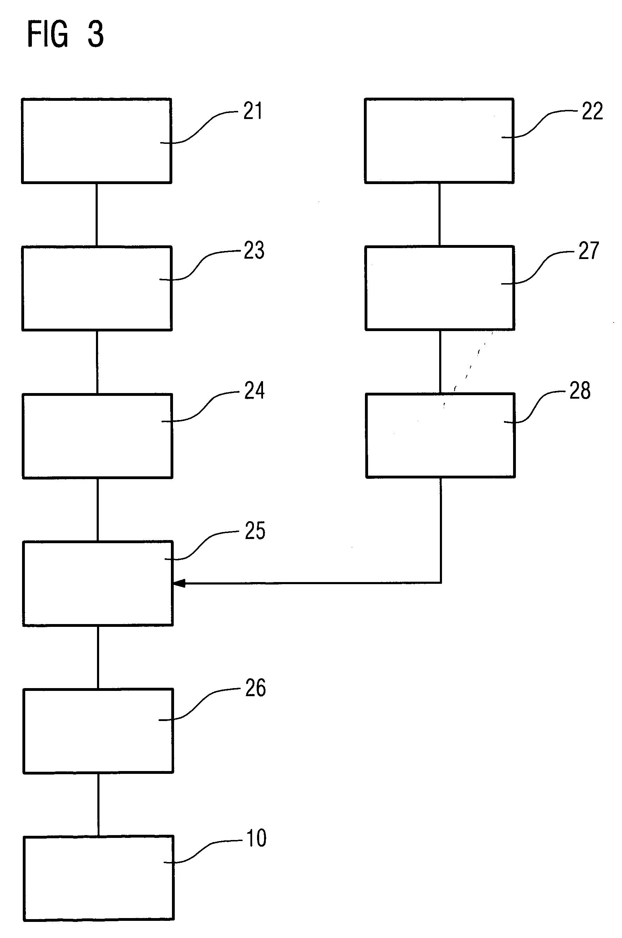

Das Lokalisierungsverfahren wird beispielhaft anhand von Figur 3 erläutert. Zunächst werden seine Eingabegrößen, d.h. die Mess- und die Modelldaten, und dann die Rechenschritte des Verfahrens erläutert.

Die Eingabegrößen für das Lokalisierungsverfahren sind pro Messfläche

a) Eine $M \times N$ Datenmatrix $D$ mit Messwerten (Bezugszeichen 21), welche von den $M$ Sensororten $\vec{r}_{s,m}$, ($m = 1,...,M$) und den $N$ Anregungsparametern ($N_1$ Anregungsorte $\vec{r}_{A,n_1}$, ($n_1 = 1,...,N_1$) und/oder $N_2$ Anregungsmodulationsfrequenzen $f_{n_2}$, ($n_2 = 1,...,N_2$), wobei $N = N_1 + N_2$), abhängt, und welche sich ggfs. durch Nachverarbeitung aus den eigentlichen Messdaten ergeben kann,

Die M-dimensionalen Spaltenvektoren der Datenmatrix lassen sich entsprechend der Anordnung der Sensoren auf der Messfläche reformatieren. Eine graphische Darstellung des reformierten Spaltenvektors visualisiert die Messwertverteilung über der betrachteten Messfläche zu gegebener Anregungsart. Im Falle oben erwähnter 8x8 Sensorverteilung wird der 64-dimensionale Spaltenvektor in eine $8 \times 8$-Matrix umgeformt.

- von optischen Parametern wie den Absorptions- und Streukoeffizienten $\mu_a, \mu_s$ des die Läsion(en) umgebenden Mediums.

b) Ein Satz von K Führungsfeldern oder Leadfields $L_k(\vec{r}_m, \vec{n}_m, \vec{r}_i, \mu_a, \mu_s)$, ($k = 1,...,K$), beispielsweise Multipol-Leadfields, welche in Figur 3 mit dem Bezugszeichen 22 gekennzeichnet sind und welche ihrerseits abhängen

- vom Modell des optischen Mediums des Untersuchungsgebiets 1,
- vom Messsystem, beispielsweise Ort $\vec{r}_m$ und/oder Normalenvektor $\vec{n}_m$ des $m$-ten Sensors,
- vom Ort $\vec{r}_f$ des $f$-ten anregbaren Fluorochroms,
- von der Art der Messung (Frequenzmodulation ja/nein) und
- von optischen Parametern wie den Absorptions- und Streukoeffizienten $\mu_a,\mu_s$ des die Läsion(en) umgebenden Mediums

Die auf einen Sensor auftreffenden Photonen werden in elektrische Signale umgewandelt und dann der weiteren Auswertung zugeführt. Im Falle frequenzmodulierter Anregung werden Lichtintensität und Phasenverschiebungen gegenüber der Eingangswelle gemessen. Beide reellen Messwerte können zu einem komplexen Messwert zusammengefasst werden. Die Datenmatrix ist dann - im mathematischen Sinne - komplex. Im Folgenden wird vom allgemeinen Fall einer komplexen Datenmatrix ausgegangen.

Es kann erforderlich sein, nachverarbeitete Messdaten dem Lokalisierungsalgorithmus zuzuführen. Beispielsweise werden durch Abschneiden von Randdaten Randartefakte eliminiert. Sie könnten eine nicht-existente Abhängigkeit von der Modulationsfrequenz- bzw. dem Anregungsort vortäuschen.

Die Datenmatrix kann sich auch aus einer Linearkombination von mindestens zwei Datensätzen ergeben. Beispielsweise kann die Differenz eines Datensatzes mit Fluoreszenzsignalen und eines räumlich benachbarten Datensatzes ohne Fluoreszenzsignal betrachtet werden. Es ist zu erwarten, dass mögliche Beiträge von Hintergrundanregungen in den Differenzdaten deutlich reduziert, wenn nicht gänzlich eliminiert sind.

Führungsfelder oder sogenannte Leadfields sind aus dem Bioelektromagnetismus bekannte Größen. Sie geben die Messwertverteilung einer Einheitssignalquelle an, die mit einem gegebenen Messsystem aufgenommen werden kann.

Leadfields, welche die mit einem Messsystem oder mehreren Messsystemen aufnehmbare Lichtintensität auf Grund optisch angeregter, fokaler und mit Fluorochromen markierten Läsionen beschreiben, sind als Eingangsgrößen für das Verfahren zur Lokalisierung solcher fokaler Läsionen geeignet.

Beispielhaft wird im Ausführungsbeispiel lediglich ein Leadfield verwendet. Es beschreibt die mit einem gegebenen Messsystem messbare Lichtintensität einer punktförmigen Lichtquelle. Räumlich ausgedehnte Fluoreszenzquellen können entsprechend den in B. Scholz, "Towards Virtual Electrical Breast Biopsy: Space-Frequency MUSIC for Trans-Admittance Data", IEEE Trans. Med. Imag., Vol. 21, No. 6, pp. 588-595, 2002, behandelten ausgedehnten elektrisch-polarisierten Läsionsgebiete ebenfalls durch Multipol-Leadfields erfasst werden. Im folgenden wird angenommen, dass beispielhaft Multipol-Leadfields, d.h. ein Satz von mehreren Leadfields, zur Verfügung stehen.

Für die weiteren Schritte ist es hilfreich, die Werte des $k$-ten Leadfields $L_k(k = 1,...,K)$ an den $M$ Messorten zu einem $M$-dimensionalen Vektor im Datenraum (symbolisiert durch den Unterstrich unter $L$) zusammenzufassen.

$$(1) \qquad \underline{L}_k(\vec{r}) \equiv \left(L_k(\vec{r},\vec{r}_1),\ldots,L_k(\vec{r},\vec{r}_M)\right)^T \quad \text{mit} \quad k = 1,\ldots,K$$

Hierbei ist $\vec{r}$ der Schwerpunktsort der Läsion. Der Übersichtlichkeit halber ist in Gleichung (1) die Abhängigkeit von den optischen Parametern des die Läsion(en) umgebenden Mediums nicht aufgeführt.

Die optischen Parameter, welche wie oben erwähnt in die Leadfields eingehen, können durch Referenzmessungen bei nicht-fluoreszenzanregenden Wellenlängen mittels Schätzverfahren bestimmt werden.

Die Signalverarbeitung des Verfahrens besteht pro Messfläche aus

1. der Singulärwertzerlegung der Datenmatrix $D$ (Bezugszeichen 23 in Figur 3),
2. der Analyse der Singulärwertzerlegung (Bezugszeichen 24 in Figur 3) und
3. dem eigentlichen Lokalisierungsverfahren (Bezugszeichen 25 in Figur 3).

Die Singulärwertzerlegung 28 einer Matrix ist eine aus G. Golub, Ch. Van Loan, *Matrix Computations,* 3rd edition, J. Hopkins University Press, 1996, Seite 70 ff., bekannte mathematische Methode. Sie lautet für obige Datenmatrix

$$(2) \qquad D = USV^H.$$

Hierbei bezeichnet

**U**  eine nur von den Indizes der Sensororte abhängige, unitäre $M \times M$ Matrix,
**S**  die $M \times N$ Singulärwert-Matrix mit *min(M,N)* reellen Singulärwerten in der Diagonalen und sonst verschwindenden Elementen und
**V**  eine nur von den Anregungsorts- bzw. Frequenzindizes abhängige, unitäre $N \times N$ Matrix und
**H**  die hermitesche Konjugation der betreffenden Matrix.

Die Singulärwerte sind entsprechend ihrer abnehmenden numerischen Größe geordnet, d.h. es gilt

$$(3) \qquad s_1 \geq s_2 \geq ... \geq s_{\min(M,N)}$$

Bezeichnet man mit $\underline{u}_q, \underline{\underline{v}}_q$ die q-ten Spaltenvektoren der Matrizes **U** und **V**, dann zeigt die alternative tensorielle Schreibweise ($\otimes$ bezeichnet das Tensorprodukt)

$$(4) \qquad \boldsymbol{D} = \sum_{q=1}^{\min(M,N)} s_q \ \underline{u}_q \otimes \underline{\underline{v}}_q^H$$

deutlich, dass der q-te Singulärwert ausschließlich mit den q-ten Spaltenvektoren von **U** und **V** verknüpft ist. Der einfache und der doppelte Unterstrich bei *u* und *v* sollen andeuten, dass es sich um einen *M*- bzw. *N*-dimensionalen Vektor handelt.
Die *M* Indizes der Spaltenvektoren $\underline{u}_q$ entsprechen den fortlaufend nummerierten Indizes der Messsensoren. Demzufolge können diese Spaltenvektoren - wie oben erwähnt - entsprechend der Anordnung der Messsensoren in Matrizes umgeformt und als zweidimensionale Messwertverteilungen dargestellt werden. Diese Spaltenvektoren sind anregungs- bzw. frequenzunabhängige orthonormierte Basisvektoren im M-dimensionalen Datenraum und werden hier als Basis- bzw. Eigenmaps bezeichnet.
Zur Singulärwertanalyse wird die Zahl $Q_{dom}$ der signifikanten Singulärwerte ermittelt, die die Zahl der sich bzgl. der Anregungsart linear unabhängig verhaltenden Fluoreszenzquellen angibt.
Eine punktförmige Inhomogenität im ansonsten homogenen optischen Medium erzeugt beispielsweise ein Singulärwertspektrum mit einem signifikanten Singulärwert ($Q_{dom}$ = 1).
Die zugehörigen Spaltenvektoren $\underline{u}_q$ werden als Basisvektoren eines - frequenzunabhängigen - $Q_{dom}$-dimensionalen Signalraumes im M-dimensionalen Datenraum betrachtet. Die restlichen M-$Q_{dom}$ Spaltenvektoren sind dann die Basisvektoren des orthogonalen Signalraumes.
Das Aufsuchen von Fuorochrom-markierten Läsionen, die Lokalisierung, entspricht der Suche von Orten bzw. Schwerpunktsorten angeregter Signalquellen. Diese Suche mittels eines Computers verlangt die Diskretisierung des angenommenen Modellmediums, welche die zu untersuchende Körperregion mathematisch nachbilden soll.
Eine Suchstrategie besteht darin, mit den anregungs- und frequenzunabhängigen Leadfields an jedem Rasterort anregungsund frequenzunabhängige Modelldaten und/oder einen Modelldatenraum zu erzeugen und diese und/oder diesen mit dem aus den Messdaten gewonnenen anregungs- und frequenzunabhängigen Signalraum zu vergleichen. Vergleichsmaße lassen sich so definieren, dass sie den Grad der "Übereinstimmung" zwischen Signalraum und Modelldaten / Modelldatenraum anzeigen. Orte, an denen das Maß ein lokales Maximum annimmt, werden als Orte tatsächlicher Signalquellen angesehen.
Eine alternative, zweite Suchstrategie besteht im Vergleich zwischen dem orthogonalen Signalraum - in der älteren Literatur auch Rauschraum genannt - und den Modelldaten bzw. dem Modelldatenraum. Vergleichsmaße lassen sich dann so definieren, dass sie den Grad der "Nicht-Übereinstimmung" zwischen dem orthogonalen Signalraum und Modelldaten / Modelldatenraum anzeigen. Orte, an denen das Maß ein lokales Minimum annimmt, werden als Orte tatsächlicher Signalquellen angesehen.
Die Modelldaten sind durch die Leadfields gegeben: entweder werden sie direkt oder nachverarbeitet genutzt.
Ein einzelnes Leadfield stellt ein Modelldatensatz dar, der eine spezielle Eigenschaft der Signalquelle widerspiegelt. Beispielsweise beschreibt das Leadfield einer punktförmigen Fluoreszenzquelle, die messbare Lichtintensität bei isotroper Lichtemission durch diese Quelle.
Die Gesamtheit der betrachteten Leadfields (Anzahl: K) definiert auf Grund ihrer linearen Unabhängigkeit einen K-dimensionalen Modelldatenraum. Die Leadfields sind i.a. nichtorthogonale Basisvektoren dieses Modelldatenraumes. Orthogonale Basisvektoren lassen sich durch geeignete Orthogonalisierungsverfahren, d.h. durch Nachverarbeitung der Leadfields, gewinnen. Sie verändern den Modelldatenraum nicht. Jedoch ergeben sich mit den neuen Basisvektoren neue einzelne Modelldatensätze (s. oben). Diese Basisvektoren können zusätzlich normiert werden. Dies stellt sicher, dass Leadfields mit unterschiedlichem Abstandsverhalten in gleicher Weise zur Lokalisierung beitragen können. Zudem hat es den Vorteil, physikalisch dimensionslose Größen zu betrachten.

Eine vorteilhafte Leadfield-Nachverarbeitung besteht beispielsweise darin, die $K$ Leadfields $\underline{L}_k$ ($k=1,...,K$) aus Gleichung (1) zu normieren (Verarbeitungsschritt 27). Dabei werden jeweils die einzelnen Führungsfelder auf ihre Norm bezogen, so dass sich die normierten Führungsfelder $\underline{L}_k^{(n)}$ wie folgt ergeben:

$$(4a) \qquad \underline{L}_k^{|n|} = \frac{\underline{L}_k}{|\underline{L}_k|}$$

Mittels beispielsweise einer Singulärwertzerlegung der $M \times K$ Leadfields-Matrix $\boldsymbol{L}$ orthogonalisierte Leadfields zu gewinnen. Die Normierung ist durch den Index $(n)$ angezeigt.

$$(5) \qquad L^{(n)} = \left| \underline{L}_1^{|n|},...,\underline{L}_K^{|n|} \right| = U_L \, S_L \, V_L^T$$

Der Übersichtlichkeit halber wurden die Argumente der Leadfields, die Ortsvektoren des Quellortes, weggelassen. Die ersten $K$ Spaltenvektoren $\underline{U}(\vec{r})_{L,k}$ ,($k = 1,...,K$) der Matrix $U_L$ sind die gesuchten quellortsabhängigen orthonormierten Leadfields. Im Falle eines einzigen Leadfields entfällt die Singulärwertzerlegung aus Gleichung (5).

Beispiele für Vergleichsmaße zwischen einem Modelldatensatz bzw. dem Modelldatenraum und dem Signalbzw. dem orthogonalen Signalraum sind aus anderen biomedizinischen Anwendungen, Analyse biomagnetischer Daten oder Analyse elektrischer Trans-Admittanzdaten, bekannt. Solche Verfahren sind Projektionsverfahren und Winkelabstandsverfahren.

Mit Hilfe von Projektionsmatrizes werden einzelne Modelldatensätze bzw. der Modelldatenraum entweder auf den Signalraum bzw. auf den orthogonalen Signalraum projiziert und für jeden Rasterort der entsprechende Projektionswert bestimmt.

Basierend auf dem in G. Golub et al. Seite 584 ff. angegebenen Algorithmus zur Berechnung von Winkeln zwischen zwei Unterräumen, dem sogenannten Winkelverfahren, werden Suchort für Suchort die Winkel zwischen dem Signalraum bzw. dem orthogonalen Signalraum und einzelnen Modelldatensätzen bzw. dem Modelldatemraum berechnet. Hier gibt ein kleiner Winkel, also ein kleiner Wert des Vergleichsmaßes, zwischen beispielsweise dem Signalraum und dem Modelldatenraum eine große "Übereinstimmung" an. Eine Transformation des Vergleichsmaßes in der Form 90 Grad - berechneter Winkel ergibt dann wieder Maxima der Vergleichsfunktion an den Orten tatsächlicher Signalquellen. Mutatis mutandis lässt sich das Gesagte auf Winkelvergleichsmaße zwischen anderen Unterräumen übertragen.

An jedem Ort $\vec{r}$ des diskretisierten optischen Modellmediums wird überprüft, wie groß der Abstand zwischen den orthogonalisierten Leadfields $\underline{U}(\vec{r})_{L,k}$ und dem Signalraum ist. Ein geeignetes Maß ist die Funktion

$$(6) \qquad F_k(\vec{r}) = [\sum_{i=1}^{Q_{dom}} c_i \underline{u}_i - \underline{U}_{L,k}]^2$$

Die Ausgangsgleichung von (6) ist die im Quadratmittelsinne zu betrachtende Gleichung

$$(7) \qquad \sum_{i=1}^{Q_{dom}} c_i \underline{u}_i = \underline{U}_{L,k} \qquad k = 1,...,K \; .$$

Wird die Lösung für die Koeffizienten $c_i$ in das Bewertungsmaß eingesetzt, dann folgt

$$(8) \qquad F_k(\vec{r}) = 1 - \sum_{i=1}^{Q_{dom}} (\underline{u}_i^H \cdot \underline{U}(\vec{r})_{L,k})^2 \; .$$

Dieses Maß entspricht einer Projektion des betrachteten Leadfields auf den orthogonalen Signalraum. Unter Benutzung der auf den orthogonalen Signalraum projizierenden Projektionsmatrix

$$(9) \qquad P_{OS} = 1 - \sum \underline{u}_i \otimes \underline{u}_i^H$$

ergibt sich

$$(10) \qquad F_k(\vec{r}) = |P_{OS}\, U(\vec{r})_{L,k}|^2$$

Die tatsächliche Lokalisierungsfunktion $F$ ist der Minimalwert der Abstände $F_k$. Sie ist definiert durch

$$(11) \qquad F(\tilde{r}) = \min_k \{F_k(\tilde{r})\}$$

Die lokalen Minima der Lokalisierungsfunktion werden entsprechend ihrer Zahlenwerte monoton aufsteigend geordnet. Die Orte, welche den ersten $Q_{dom}$ lokalen Minima zuzuordnen sind, werden als Orte von Signalgeneratoren angesehen.

Im Falle mehrerer ($M_{sys}$) Messflächen werden die obengenannten Rechenschritte für die Daten jeder Messfläche separat ausgeführt. Pro Messfläche ergibt sich dann eine Zielfunktion gemäß Gleichung (11). Aus diesen Einzelzielfunktionen lässt sich eine Gesamtzielfunktion $F^{(gesamt)}$ gemäß

$$(12) \qquad F^{(gesamt)}(\vec{r}) = \sum_{\mu=1}^{M_{sys}} F^{(\mu)}(\vec{r})$$

definieren. Hierbei ist $F^{(\mu)}$ die Zielfunktion der μ-ten Messfläche.

Die lokalen Minima der Gesamt-Lokalisierungsfunktion werden wie oben entsprechend ihrer Zahlenwerte monoton aufsteigend geordnet. Die Orte, welche den ersten $Q_{dom}$ lokalen Minima zuzuordnen sind, werden als Orte von Signalquellen angesehen. Das Ausführungsbeispiel bestätigt die Erwartung, dass bei mehreren nichttrivial angeordneten Messflächen die lokalen Minima der Einzelzielfunktionen deutlicher ausgeprägt sind und damit das Lokalisierungsergebnis sicherer machen.

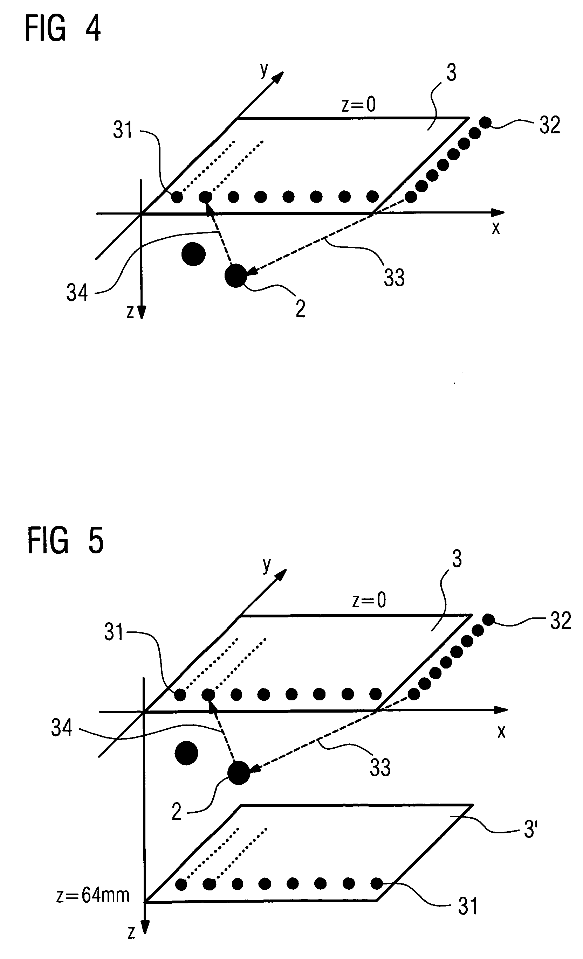

Die Aufführungsbeispiele wurden mit planaren, in dem Applikator 3 angeordneten Messsystemen gewonnen, welche 8x8 regulär angeordnete Photosensoren 31 enthielten, wie sie in Figur 4 schematisch dargestellt sind. Die Sensoren 31 wurden punktförmig angenommen. Ihr Abstand längs einer Richtung betrug 8 mm, so dass sich eine Messfeldfläche 56x56 mm$^2$ ergibt. Die Orte, an denen sich 8 Laserdioden 32 befinden, welche das Fluoreszenz anregende NIR-Licht in die Körperregion einstrahlen, können beispielsweise neben der Messfläche angeordnet sein. Die Anregung kann, muss aber nicht, frequenzmoduliert sein. Eine solche Messanordnung kann mit der Hand über einen interessierenden Gewebeabschnitt 1 geführt werden. Die Laserdioden 32 senden Anregungsstrahlen 33 aus, die auf das fluoreszierende Raumgebiet 2 treffen. Die Fluoreszensstrahlen 34 werden von den Photosensoren 31 erfasst.

In Figur 5 ist ein Doppelssytem eines Applikators 3 mit zwei gegenüberliegenden planare Messflächen gleicher Dimensionierung (8x8 Sensoren) dargestellt. Sie können beispielsweise in den Anpressplatten eines Röntgenmammographiegerätes integriert sein. Die Fluoreszenzanregung erfolgt an 8 Anregungsorten, welche sich neben der Messfläche (z=0) des oberen Applikators 3 befinden. Der Abstand der beiden Applikatoren 3 beträgt 64 mm.

Als optische Gewebemodelle werden bei den vorliegenden Beispielen folgende Modelle herangezogen:

A) Das einfachste Modell ist ein berandungsloses Gebiet mit punktförmigen fluoreszierenden Objekten, welches ansonsten optisch homogen (konstante optische Parameter wie Absorptions- und Streukoeffizient) ist.

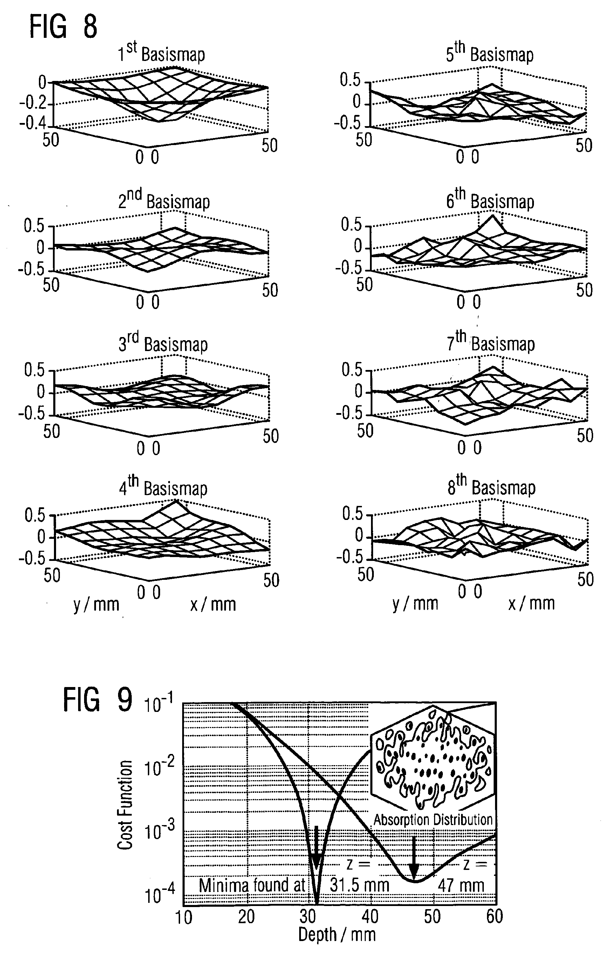

B) Als zweites Modell wurde ein optisch inhomogener Quader Gebiet mit punktförmigen fluoreszierenden Objekten betrachtet. Es wurde angenommen, dass Absorptions- und Streukoeffizient lokal um 100% variieren können.Die Figur 9 zeigt die Lokalisierungsfunktionen von 32 mm und 48 mm tiefen, Fluorochrom-markierten Läsionen. Der Absorptionskonstrastunterschied des umgebenden Gewebes beträgt 100% (Bild in Bild).

Der Simulation der Daten werden folgende Konfigurationen zugrunde gelegt:

| Konfiguration 1 | |
| --- | --- |
| Mess-/Anregungssystem | siehe Figur 4, die Anregung ist nicht frequenzmoduliert |
| Gewebemodell | inhomogener Quader (5.2.B) |
| Fluoreszenzquelle | Ort bei (x,y,z)=(28,28,32) mm, d.h. zentrale Lage unterhalb der Messfläche in einer Tiefe von 32 mm (Koordinatensystem s. Figur 4) |
| Daten | siehe Figur 6 |
| Konfiguration 2 | |
| Mess-/Anregungssystem | siehe Figur 4, die Anregung ist nicht frequenzmoduliert |
| Gewebemodell | inhomogener Quader (5.2.B) |
| Fluoreszenzquelle | Ort bei (x,y,z)=(28,28,48) mm, d.h. zentrale Lage unterhalb der Messfläche in einer Tiefe von 48 mm (Koordinatensystem s. Figur 4) |
| Konfiguration 3a,4a und 5a | |
| Mess-/Anregungssystem | Einzelmesssystem. siehe Figur 4, die Anregung ist nicht frequenz-moduliert |
| Gewebemodell | homogenes, unberandetes Medium (5.2.A) |
| Einzel-Fluoreszenzquellen | Orte bei (x,y,z)=(28,28,16) mm, (28,28,32) mm, (28,28,48) mm d.h. zentrale Lagen unterhalb der Messfläche in Tiefen von 16 mm, 32 mm und 48 mm (Koordinatensys-tem s. Figur 4) |
| Konfiguration 3b,4b und 5b | |
| Mess-/Anregungssystem | Doppelmessystem, siehe Figur 5, die Anregung ist nicht frequenz-moduliert |
| Gewebemodell | homogenes, unberandetes Medium (5.2.A) |
| Einzel-Fluoreszenzquellen | Orte bei (x,y,z)=(28,28,16) mm, (28,28,32) mm, (28,28,48) mm d.h. zentrale Lagen unterhalb der Messfläche in Tiefen von 16 mm, 32 mm und 48 mm (Koordinatensystems. Figur 5) |

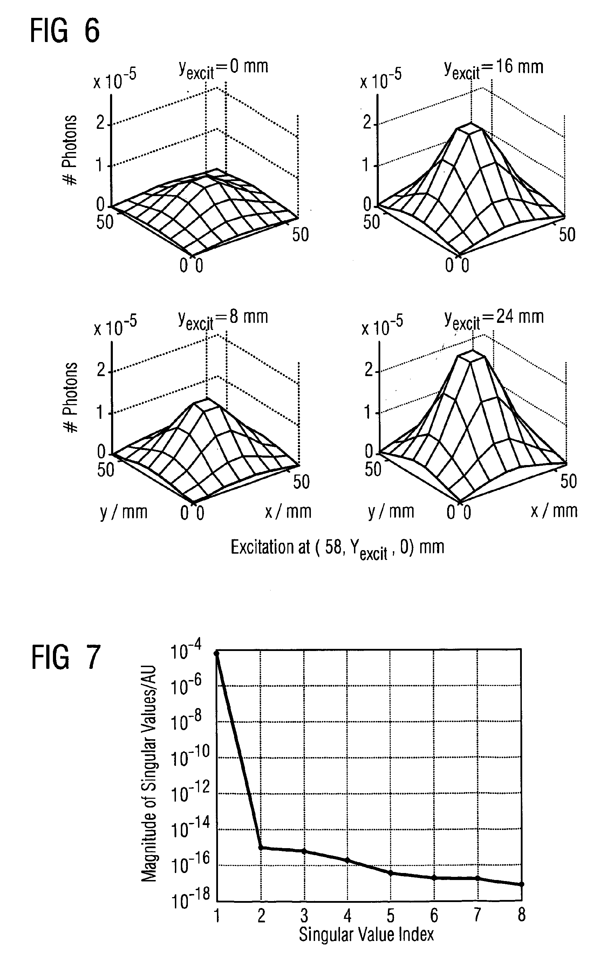

Das in Figur 7 dargestellte Singulärwertspektrum der Daten der Konfiguration 1 aufgrund der Singulärwertzerlegung 23 weist erwartungsgemäß - entsprechend der Zahl der vorhandenen Fluoreszenzquellen - einen numerisch dominanten Singulärwert auf. Die restlichen Singulärwerte geben Rauschen, in diesem Fall numerisches Rauschen, wieder.

Die zugehörigen Basismaps bzw. Eigenmaps zeigt die Figur 8. Entsprechend dem einzigen numerisch dominanten Singulärwert gibt es eine strukturierte Basismap. Sie definiert den hier eindimensionalen Signalraum des (hier 64-dimensionalen) Datenraumes.

Die oben definierten Ziel funktionen zur Lokalisierung 25, d.h. die Lokalisierungsfunktionen, der Konfigurationen 1 und 2 sind in Figur 9 dargestellt.

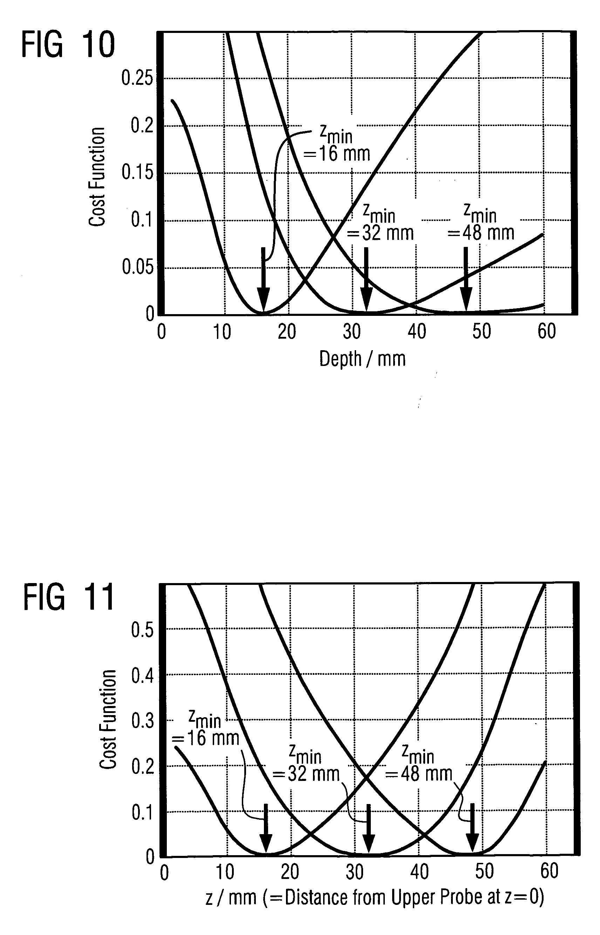

Der Einfluss einer zweiten gegenüberliegenden Messfläche auf die Lokalisierung wird an Hand der Ziel funktionen der Konfigurationen 3 a/b, 4 a/b und 5 a/b gezeigt, wobei Figur 10 die Lokalisierung unterschiedlich tiefer Läsionen mit einem planaren Messsystem und Figur 11 die Lokalisierung unterschiedlich tiefer Läsionen mit zwei gegenüberliegenden planaren Messsystemen zeigen. Die Lagen der Messsonden sind durch dicke Striche an dem linken Rand bzw. an beiden seitlichen Rändern markiert. Gegenüber Figur 10 ist eine deutlichere Ausprägung der Minima sichtbar, wobei zu beachten ist, dass die Skala gemäß Figur 11 gegenüber der von Figur 10 unterschiedlich ist.

Durch das erfindungsgemäße Verfahren kann das Problem der Lokalisierung fluoreszierender Objekte in optisch trüben Medien schnell gelöst werden. Weiterhin wird durch die Variation des Anregungsortes die Genauigkeit erhöht.

Dieses Lokalisationsverfahren zeichnet sich dadurch aus, dass es

- in Echtzeit arbeitet,
- patientenunabhängig ist und
- robust gegen Schätzung optischer Parameter ist.

**Patentansprüche**

1. Verfahren zur Lokalisierung von Bereichen (2) in einem biologischen Gewebeabschnitt (1), die zumindest während der Untersuchung eine vom Gewebeabschnitt (1) verschiedene Fluoreszenzeigenschaft aufweisen, aufgrund derer bei einer Bestrahlung mit Licht (33) einer ersten Wellenlänge Licht (34) einer anderen Wellenlänge emittiert wird, mit den Schritten:

   a) Anlegen einer Folge von fluoreszenzanregenden Lichtsignalen an unterschiedlichen Orten auf dem Gewebeabschnitt (1),
   b) Messen von Fluoreszenzlicht (21) an mehreren Messorten auf einer Oberfläche des Gewebeabschnittes (1), die sich aufgrund der Lichtsignale dort einstellen,
   c) Bestimmen von frequenzunabhängigen Signalanteilen in den Antwortsignalen und Weiterverarbeitung (23, 24) der frequenzunabhängigen Signalanteile zu Eingabewerten eines Lokalisationsschritts (25),
   d) Modellieren des Gewebeabschnitts (1) und Bestimmen eines Satzes von Führungsfeldern (22),
   e) Transformierung (28) der Führungsfelder (22), so dass im Lokalisierungsschritt (25) die frequenzunabhängigen Signalanteile mit den transformierten Führungsfeldern verglichen werden und dass der Ort (26) der transformierten Führungsfelder, die die frequenzunabhängigen Signalanteile am besten wiedergeben, als Ort des zu lokalisierenden Bereichs (2) ausgegeben wird.

2. Verfahren nach Anspruch 1, **dadurch gekennzeichnet, dass** zur Erzeugung der verschiedenen Fluoreszenzeigenschaften die Bereiche (2) mit fluoreszierenden Markern (Fluorophore) markiert werden.

3. Verfahren nach Anspruch 1 oder 2, **dadurch gekennzeichnet, dass** die fluoreszenzanregenden Lichtsignalen mit verschiedenen Modulationsfrequenzen erzeugt und in den Gewebeabschnitt (1) eingestrahlt werden.

4. Verfahren nach einem der Ansprüche 1 bis 3, **dadurch gekennzeichnet, dass** die fluoreszenzanregenden Lichtsignalen durch Laserlicht (33) geeigneter Wellenlänge eingestrahlt werden.

5. Verfahren nach einem der Ansprüche 1 bis 4, **dadurch gekennzeichnet, dass** die Führungsfelder (22) zunächst normiert (27) und dann transformiert (28) werden.

6. Verfahren nach einem der Ansprüche 1 bis 5, **dadurch gekennzeichnet, dass** die Führungsfelder zu orthogonalen Führungsfeldern transformiert (28) werden.

7. Verfahren nach Anspruch 6, **dadurch gekennzeichnet, dass** die orthogonalen Führungsfelder mittels einer Singulärwertzerlegung (28) aus den Führungsfeldern (22) bestimmt werden.

8. Verfahren nach Anspruch 7, **dadurch gekennzeichnet, dass** die optischen Parameter durch Referenzmessungen bei nicht-fluoreszenzanregenden Wellenlängen mittels Schätzverfahren bestimmt werden.

9. Vorrichtung zur Durchführung des Verfahrens nach einem der Ansprüche 1 bis 8, **gekennzeichnet durch,** dass wenigstens eine Anordnung (3) von auf der Oberfläche des Gewebeabschnitts (1) verteilter Lichtsensoren (31) zur Messung des von dem fluoreszierend markierten Bereich (2) emittierten Fluoreszenzlichtes (34) vorgesehen ist, neben denen Laserdioden (32) zur Erzeugung von den fluoreszierend markierten Bereich (2) anregendem Licht (33), um somit von der Art der Anregung abhängende zweidimensionale Messwertverteilungen zu erhalten.

10. Vorrichtung nach Anspruch 9, **dadurch gekennzeichnet, dass** zwei Anordnungen (3) von Lichtsensoren (31) vorgesehen sind, die beidseitig des zu untersuchenden Gewebeabschnittes (1) anlegbar sind.

11. Vorrichtung nach Anspruch 8 oder 10, **dadurch gekennzeichnet, dass** die Anordnungen (3) in Anpressplatten eines Röntgen-Mammographie-Gerätes integriert sind.

12. Vorrichtung nach einem der Ansprüche 8 bis 11, **dadurch gekennzeichnet, dass** die Anordnungen (3) flexibel ausgeführt sind.

13. Vorrichtung nach einem der Ansprüche 8 bis 12, **dadurch gekennzeichnet, dass** die Anordnungen (3) gekrümmt ausgeführt sind.

FIG 1

FIG 2

## FIG 3

## FIG 4

## FIG 5

# FIG 6

Excitation at ( 58, $Y_{excit}$ , 0) mm

# FIG 7

# FIG 8

1st Basismap

5th Basismap

2nd Basismap

6th Basismap

3rd Basismap

7th Basismap

4th Basismap

y / mm          x / mm

8th Basismap

y / mm          x / mm

# FIG 9

Cost Function

Absorption Distribution

Minima found at    31.5 mm        47 mm

z =                z =

Depth / mm

FIG 10

Cost Function

0.25

0.2

$z_{min}$
$=16$ mm

0.15

0.1

$z_{min}$
$=32$ mm

$z_{min}$
$=48$ mm

0.05

0

0    10    20    30    40    50    60

Depth / mm

FIG 11

Cost Function

0.5

0.4

$z_{min}$
$=16$ mm

0.3

$z_{min}$
$=32$ mm

$z_{min}$
$=48$ mm

0.2

0.1

0

0    10    20    30    40    50    60

z / mm (=Distance from Upper Probe at z=0)

# EP 1 421 895 A1

EUROPÄISCHER TEILRECHERCHENBERICHT

Europäisches Patentamt

der nach Regel 45 des Europäischen Patent-übereinkommens für das weitere Verfahren als europäischer Recherchenbericht gilt

Nummer der Anmeldung

EP 03 02 3889

## EINSCHLÄGIGE DOKUMENTE

| Kategorie | Kennzeichnung des Dokuments mit Angabe, soweit erforderlich der maßgeblichen Teile | Betrifft Anspruch | KLASSIFIKATION DER ANMELDUNG (Int.Cl.7) |
|---|---|---|---|
| D,A | SCHOLZ B: "TOWARDS VIRTUAL ELECTRICAL BREAST BIOPSY: SPACE-FREQUENCY MUSIC FOR TRANS-ADMITTANCE DATA" IEEE TRANSACTIONS ON MEDICAL IMAGING, IEEE INC. NEW YORK, US, Bd. 21, Nr. 6, Juni 2002 (2002-06), Seiten 588-595, XP001124745 ISSN: 0278-0062 * das ganze Dokument * --- | 9-13 | A61B5/00 |
| D,A | WO 02 41760 A (NTZIACHRISTOS VASILIS ;GEN HOSPITAL (US); WEISSLEDER RALPH (US)) 30. Mai 2002 (2002-05-30) * Seite 3, Zeile 11 - Seite 4, Zeile 6 * * Seite 20, Zeile 6-17 * * Seite 21, Zeile 9-22; Abbildungen 3A-3F,5 * --- | 9-13 | |
| A | US 5 931 789 A (CAI WEI ET AL) 3. August 1999 (1999-08-03) * Spalte 3, Zeile 38 - Spalte 5, Zeile 11 * --- -/-- | 9-13 | **RECHERCHIERTE SACHGEBIETE (Int.Cl.7)** A61B G01N |

## UNVOLLSTÄNDIGE RECHERCHE

Die Recherchenabteilung ist der Auffassung, daß ein oder mehrere Ansprüche, den Vorschriften des EPÜ in einem solchen Umfang nicht entspricht bzw. entsprechen, daß sinnvolle Ermittlungen über den Stand der Technik für diese Ansprüche nicht, bzw. nur teilweise, möglich sind.

Vollständig recherchierte Patentansprüche:

Unvollständig recherchierte Patentansprüche:

1-8

Nicht recherchierte Patentansprüche:

Grund für die Beschränkung der Recherche:

Artikel 52 (4) EPÜ - Verfahren zur chirurgischen Behandlung des menschlichen oder tierischen Körpers

| Recherchenort | Abschlußdatum der Recherche | Prüfer |
|---|---|---|
| BERLIN | 31. März 2004 | Pohjamo, T |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
......................................................
& : Mitglied der gleichen Patentfamilie, übereinstimmendes Dokument

EPO FORM 1503 03.82 (P04C09)

Europäisches
Patentamt

**EUROPÄISCHER
TEILRECHERCHENBERICHT**

*Nummer der Anmeldung*

EP 03 02 3889

| EINSCHLÄGIGE DOKUMENTE | | | KLASSIFIKATION DER ANMELDUNG (Int.Cl.7) |
|---|---|---|---|
| Kategorie | Kennzeichnung des Dokuments mit Angabe, soweit erforderlich der maßgeblichen Teile | Betrifft Anspruch | |
| A | DE 101 09 539 A (SIEMENS AG) 12. September 2002 (2002-09-12) * Absätze [0002]-[0013] * * Absätze [0041]-[0054] * ----- | 9-13 | |
| | | | **RECHERCHIERTE SACHGEBIETE** (Int.Cl.7) |

EPO FORM 1503 03.82 (P04C12)

## ANHANG ZUM EUROPÄISCHEN RECHERCHENBERICHT
## ÜBER DIE EUROPÄISCHE PATENTANMELDUNG NR.

EP 03 02 3889

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

31-03-2004

| Im Recherchenbericht angeführtes Patentdokument | | Datum der Veröffentlichung | Mitglied(er) der Patentfamilie | | Datum der Veröffentlichung |
|---|---|---|---|---|---|
| WO 0241760 | A | 30-05-2002 | US | 6615063 B1 | 02-09-2003 |
| | | | AU | 2867302 A | 03-06-2002 |
| | | | CA | 2428462 A1 | 30-05-2002 |
| | | | EP | 1349490 A2 | 08-10-2003 |
| | | | WO | 0241760 A2 | 30-05-2002 |
| | | | US | 2004015062 A1 | 22-01-2004 |
| US 5931789 | A | 03-08-1999 | US | 5813988 A | 29-09-1998 |
| | | | US | 6108576 A | 22-08-2000 |
| DE 10109539 | A | 12-09-2002 | DE | 10109539 A1 | 12-09-2002 |
| | | | CA | 2438989 A1 | 06-09-2002 |
| | | | WO | 02067773 A1 | 06-09-2002 |
| | | | EP | 1363532 A1 | 26-11-2003 |

EPO FORM P0461

Für nähere Einzelheiten zu diesem Anhang : siehe Amtsblatt des Europäischen Patentamts, Nr.12/82